(19) Europäisches Patentamt
European Patent Office
Office européen des brevets

(11) Numéro de publication : **0 143 016**
**B1**

(12) **FASCICULE DE BREVET EUROPÉEN**

(45) Date de publication du fascicule du brevet : **29.07.87**

(51) Int. Cl.[4] : **C 07 D295/14**, A 61 K 31/495

(21) Numéro de dépôt : **84401824.2**

(22) Date de dépôt : **14.09.84**

(54) **Dérivés de 4-(3-alkynyloxy-2-hydroxy-propyl)-piperazin-1-yl-N-phényl acétamide, leur préparation et leur application en thérapeutique.**

(30) Priorité : **15.09.83 FR 8314720**

(43) Date de publication de la demande : **29.05.85 Bulletin 85/22**

(45) Mention de la délivrance du brevet : **29.07.87 Bulletin 87/31**

(84) Etats contractants désignés : **AT BE CH DE FR GB IT LI LU NL SE**

(56) Documents cités :
**EP-A- 0 000 299**
**FR-A- 2 173 869**

(73) Titulaire : **RIOM LABORATOIRES- C.E.R.M. "R.L.-CERM" - (S.A.)**
**Route de Marsat B.P. 140**
**F-63203 Riom Cédex (FR)**

(72) Inventeur : **Combourieu, Michel**
**6, rue du Mont Mouchet**
**F-15000 Aurillac (FR)**
Inventeur : **Laigle, Jean-Claude**
**31, Avenue Aristide Briand**
**F-15000 Aurillac (FR)**
Inventeur : **Reyjal, Jean-François**
**La Course du Mouton Roannes Saint-Mary**
**F-15220 Saint-Mamet La Salvetat (FR)**
Inventeur : **Landes, Marie-Paule**
**20, Avenue de la Liberté**
**F-15000 Aurillac (FR)**

(74) Mandataire : **Thérond, Gérard Raymond et al**
**RIOM LABORATOIRES - CERM - S.A. Route de Marsat**
**B.P. 140**
**F-63203 Riom Cédex (FR)**

**EP 0 143 016 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés d'amino-alcools et plus spécialement des dérivés de 4-(3-alkynyloxy-2-hydroxy-propyl)-piperazin-1-yl-N-phenyl acetamide de formule I

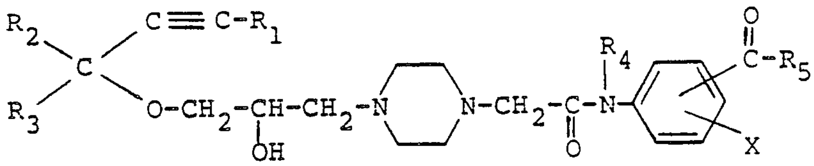

(I)

dans laquelle $R_1$ représente l'hydrogène ou un radical alkyle inférieur ; $R_2$ et $R_3$ représentent séparément le radical phényle, un radical alkyle inférieur ou ensemble avec l'atome de carbone auquel ils sont liés un radical cycloalkyle ; $R_4$ représente l'hydrogène ou un radical alkyle inférieur ; $R_5$ représente un radical alkyle inférieur ou le radical phényle éventuellement substitué par un atome d'halogène, X représente l'hydrogène ou un atome d'halogène, ainsi que leurs sels d'addition pharmaceutiquement acceptables desdits composés.

Dans la définition des substituants $R_1$ à $R_4$ un « radical alkyle inférieur » désigne un radical alkyle linéaire ou ramifié ayant 1 à 6 atomes de carbone et de préférence 1 à 4 atomes de carbone tels que les radicaux méthyle, éthyle, propyle, isopropyle, butyle, isobutyle. Selon une réalisation préférée « un radical alkyle inférieur » représente le radical méthyle.

Dans la définition de « X », un halogène signifie le fluor, le chlore, le brome ou l'iode. Selon une réalisation préférée, lorsque « X » est un halogène, il représente le chlore de préférence en position 4.

Le reste cycloalkyle dans la définition de $R_2$ et $R_3$ signifie un reste cycloaliphatique à 5, 6 ou 7 chaînons tels que les restes cyclopentyle, cyclohexyle et cycloheptyle.

Le reste

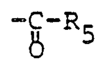

peut être fixé en position quelconque sur le radical phényl, la position préférée étant en ortho par rapport à l'atome d'azote du reste acétamide.

L'invention concerne également l'application de ces composés et de leurs sels organiques et inorganiques pharmaceutiquement acceptables en thérapeutique pour le traitement des affections angineuses.

Les composés de l'invention peuvent être préparés selon des méthodes bien connues pour la préparation de composés analogues. Par exemple, on peut utiliser un procédé correspondant au schéma réactionnel suivant :

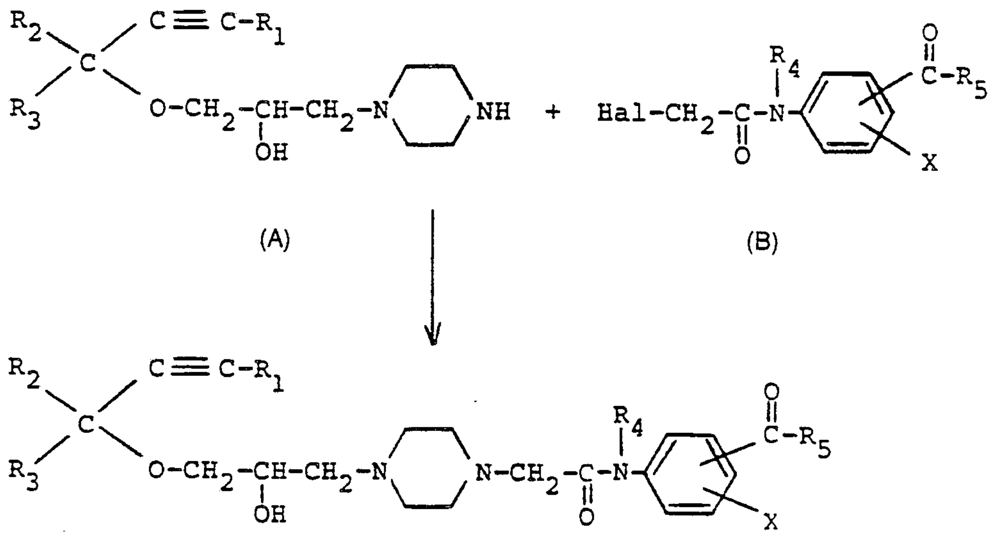

(A) (B)

« Hal » désigne un halogène, de préférence le chlore ou le brome, et $R_1$ à $R_5$ et X ont les significations précédemment indiquées. La réaction s'effectue de préférence en mettant en jeu des quantités sensiblement équimoléculaires des composés (A) ou de l'un de ses sels, et (B) en solution dans un solvant organique, en présence d'un dérivé basique à une température comprise entre la température

ambiante et la température de reflux de solvant ; le composé recherché est isolé par des moyens habituels comme l'évaporation du solvant, l'extraction à l'éther puis transformé en un sel d'amine pharmaceutiquement acceptable par action sur la base libre d'un acide organique ou inorganique, tels que les acides chlorhydrique, fumarique, acétique, maléique ou citrique.

A titre de solvant organique, on utilisera par exemple l'éthanol, le chlorure de méthylène ou le diméthylformamide. A titre de composé basique, on utilisera par exemple le carbonate de potassium ou la triéthylamine.

Pour la préparation de la matière première (A), on se reportera avantageusement à la description donnée dans le brevet FR 72 12 893. Pour la préparation de la matière première (B), on fera par exemple agir le chlorure de chloracétyle sur une amino acétophénone ou une amino benzophénone substituées ou non en présence de triéthylamine.

Selon une variante du procédé, les composés de l'invention peuvent également être préparés selon le schéma réactionnel suivant :

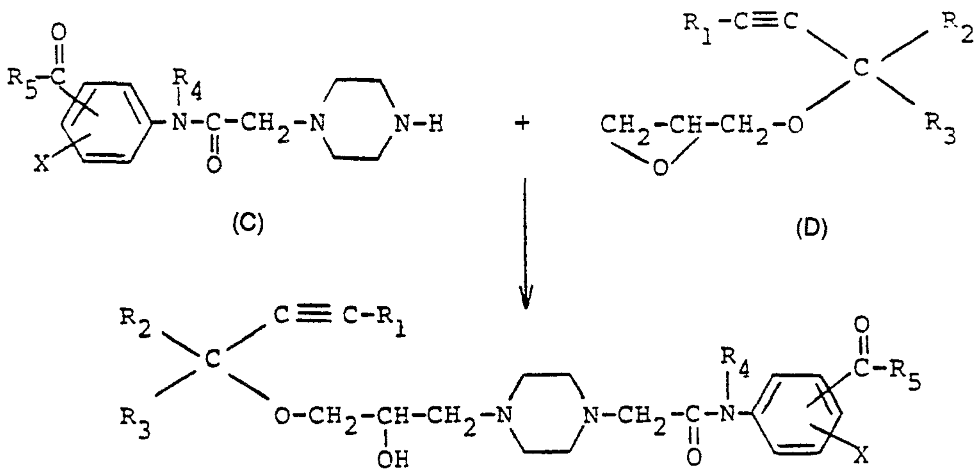
(C) (D)

(I)

Les composés (I) ainsi obtenus peuvent ensuite être transformés en sels d'addition acide pharmaceutiquement acceptables.

La matière première (C) peut être préparée selon la réaction suivante :

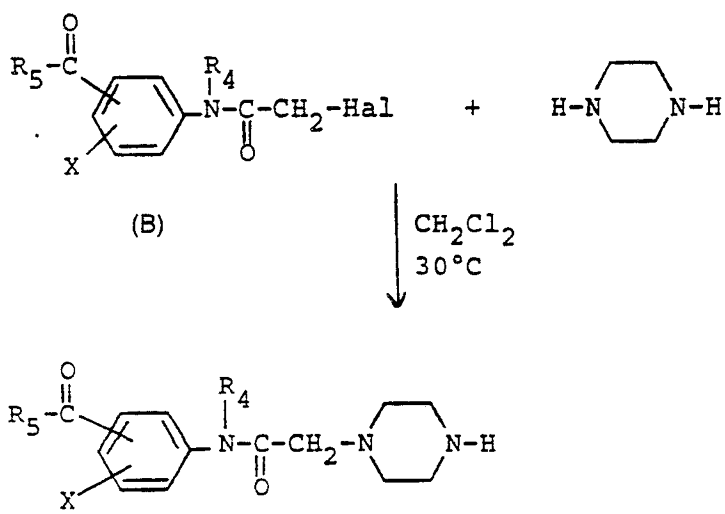

(B)

(C)

La réaction de (C) avec (D) s'effectue de préférence dans un solvant organique anhydre tel que l'alcool absolu. Pour la préparation de l'époxyde de formule (D), on se référera au brevet FR 72-12893.

L'activité anti-angineuse des composés de l'invention a été étudiée chez l'animal en recherchant leurs effets hémodynamiques sur le chien anesthésié, conformément au protocole résumé ci-après.

Chez le chien anesthésié au chloralose (100 mg · $kg^{-1}$ I.V.), les paramètres suivants sont enregistrés :

fréquence cardiaque à l'aide d'électrodes à ECG sous-cutanée reliées à un cardiotachymètre BECKMAN® (dérivation D II)

débit artériel coronaire enregistré par l'intermédiaire d'un débitmètre électromagnétique STATHAN® on étudie aussi l'action antitachycardisante en mesurant son activité inhibitrice des effets chronotropes positifs de l'isoprénaline.

Les différents paramètres sont enregistrés en continu sur un dynographe BECKMAN®, et on mesure en même temps la durée d'action.

Les composés de l'invention sont administrés par voie I.V. à la dose de 5 $mg.kg^{-1}$.

Les résultats enregistrés, exprimés sous forme de pourcentage de variation, sont résumés dans le tableau I ci-après.

Les composés de l'invention ont une faible toxicité administrés à la dose de 640 $mg \cdot kg^{-1}$ par voie orale chez la souris, on n'a observé aucun décès.

Il ressort des résultats enregistrés (voir tableau I) que les composés de l'invention, particulièrement les composés n^os 2, 4, 6 et 7, peuvent être utilisés en thérapeutique humaine en tant que médicament pour le traitement des troubles cardiovasculaires, notamment comme anti-angoreux.

Tableau I

| COMPOSE N° * | FREQUENCE CARDIAQUE | | DEBIT CORONAIRE | | ANTITACHY-CARDIE | |
|---|---|---|---|---|---|---|
| | V | D | V | D | V | D |
| 2 | - 21 | 60 | + 82 | 3 | - 60 | 60 |
| 4 | - 19 | >15 | + 64 | 1 | - 25 | 35 |
| 6 | - 17 | >45 | + 77 | 3 | - 47 | 15 |
| 7 | - 19 | >15 | + 124 | 2 | - 25 | 5 |
| 9 | - 13 | 5 | + 111 | 2 | - 19 | 5 |
| 10 | - 13 | 15 | + 91 | 0,5 | - 44 | 15 |

* voir définition tableau II

Les composés de l'invention peuvent être administrés par voie enthérale ou parenthérale, de préférence à une dose journalière comprise entre 1 et 20 mg par kg de poids corporel, selon les modes d'administration. En thérapeutique humaine, la dose préférée peut varier entre 75 et 750 mg par jour, et plus spécialement entre 75 et 500 mg par jour.

Mélangés avec des excipients convenables, les composés I ou leurs sels peuvent être comprimés sous forme de prises unitaires tels que comprimés, pilules etc. ou peuvent être mis en capsules. Au moyen de liquides convenables, les composés peuvent aussi être utilisés en préparations injectables ou orales sous forme de solutions, suspensions ou émulsions.

Les composés de formule I possèdent au moins un carbone asymétrique, de sorte qu'il est possible d'obtenir un mélange racémique de I ou les énantiomères optiques séparés. Le mélange racémique ainsi que les énantiomères optiques séparés appartiennent à la fois aux composés selon l'invention. Les énantiomères optiques séparés peuvent être préparés de manière habituelle par résolution du mélange racémique, ou directement en partant de matières premières optiquement active.

Exemple 1

4-[3-(1-(propyn-1-yl)-cyclohexyloxy)-2-hydroxy-propyl]-piperazin-1-yl-N-(2-benzoylphenyl)-acetamide

Dans un premier stade, on a préparé la 2-chloroacétamido-benzo-phénone : dans un tricol de 500 ml, on a introduit 25,9 g (0,23 M) de chlorure de chloracétyle puis on a ajouté goutte à goutte, en maintenant la température à 0 °C, un mélange de 19,7 g (0,1 M) de 2-amino benzophénone et de 21 g (0,2 M) de triéthylamine en solution dans 200 ml de chlorure de méthylène, puis on a laissé le mélange sous agitation pendant 1 h 1/2. On a alors lavé la phase chlorure de méthylène à l'acide chlorhydrique à 10 %, puis à l'eau et séché sur $Na_2SO_4$ anhydre. Après évaporation à sec, on a repris le résidu dans l'éthanol, filtré le précipité obtenu, on l'a lavé à l'éthanol puis à l'éther et séché. On a ainsi obtenu 19 g de l'amide cherchée. Dans un deuxième stade, on a dissous 4,9 g (0,018 M) de l'amide précédente dans 100 ml de diméthylformamide, puis on a ajouté 3 g (0,021 M) de carbonate de potassium et 5 g (0,018 M) de 1-[1-(propyn-1-yl)]-cyclohexyloxy-3-piperazin-1-yl propane-2-ol, et laissé le mélange sous agitation à température ambiante pendant 4 heures.

On a ensuite ajouté 50 ml d'eau, extrait deux fois à l'éther éthylique, lavé la phase éthérée à l'eau, puis l'a séché sur $Na_2SO_4$ anhydre, et évaporé le solvant à sec. On a ensuite dissous le résidu obtenu dans le

minimum d'acétate d'éthyle, puis on a ajouté 3,9 g d'acide maléique préalablement dissous dans la quantité minimale d'acétate d'éthyle. On a filtré le précipité obtenu, l'a rincé à l'acétate d'éthyle puis à l'éther, et séché sous vide à 80 °C. On a ainsi obtenu 8,8 g de composé cherché sous forme de dimaléate ayant pour point de fusion F = 108,4 °C.

Exemple 2

4[3-(1-propyn-1-yl)-cyclohexyloxy)-2-hydroxy-propyl]-piperazin-1-yl-N-methyl-N-[4-chloro-2-benzoyl-phenyl] acetamide

Dans un ballon de 1 litre sous agitation magnétique et à température ambiante, on a introduit 11,2 g (0,05 M) de 1-[1-(propyn-1-yl)-cyclohexyloxy]-3-(piperazin-1-yl)-propan-2-ol dissous dans 4,35 g (0,045 M) de triéthylamine et 15 g (0,041 M) de 2-(N-méthyl) bromoacétamido-5-chloro-benzophénone dans 250 ml de chlorure de méthylène et on a maintenu l'agitation à température ambiante pendant 16 heures.

On a ensuite lavé à l'eau, séché la phase organique sur $Na_2SO_4$ anhydre, et évaporé à sec. L'huile résiduelle a été dissoute dans 300 ml d'acétone anhydre puis on a ajouté 10,4 g (0,089 M) d'acide maléique et porté le mélange à reflux pendant 5 minutes. Après refroidissement, on a filtré le précipité obtenu, a rincé à l'acétone puis séché.

On a obtenu 18 g de composé cherché sous forme de dimaléate ayant pour point de fusion F = 123,6 °C.

Exemple 3

4-[3-(1-(propyn-1-yl)-cyclohexyloxy)-2-hydroxy-propyl]-piperazin-1-yl-N-(2-acetyl-phenyl)-acetamide

En procédant comme indiqué au premier stade de l'exemple 1, mais en partant de 25,35 g (0,225 M) de chlorure de chloracétyle, de 13,5 g (0,1 M) de 2-amino-acétophénone et de 21 g (0,2 M) de triéthylamine avec 200 ml de chlorure de méthylène comme solvant, on a obtenu 11,8 g de 2-chloroacétamido-acétophénone. Dans le deuxième stade, on a dissous dans un ballon de 250 ml, 4,2 g (0,0196 M) de l'amide précédente dans 100 ml de diméthylformamide, puis on a ajouté 3,3 g (0,023 M) de carbonate de potassium et 5,5 g (0,0196 M) de l'aminoalcool utilisé dans les exemples précédents. Après avoir laissé la réaction se poursuivre pendant 12 heures à température ambiante, on a repris par l'eau, extrait à l'éther, lavé à l'eau, séché sur $Na_2SO_4$ et évaporé à sec. On a ainsi obtenu 9,2 g de base (0,02 M) qu'on a traité par 4,7 g (0,04 M) d'acide maléique dans l'acétate d'éthyle pour obtenir 8 g du composé cherché sous forme de dimaléate ayant pour point de fusion F = 139,4 °C.

Exemple 4

4-[3-(1-ethynyl-cyclohexyloxy)-2-hydroxy-propyl]-piperazin-1-yl-N-[4-chloro-2-(2-chlorobenzoyl)-phenyl] acetamide

Dans un ballon de 500 ml placé sur un agitateur magnétique chauffant, on a introduit 12,8 g (0,033 M) de 2-bromoacétamido-2', 5-dichloro-benzophénone, 8 g de 1-(1-ethynyl-cyclohexyloxy)-3-(piperazin-1 yl)-propan-2-ol, 4,15 g (0,03 M) de carbonate de potassium et 250 ml d'éthanol absolu, puis on a porté le mélange à reflux pendant 6 heures. La réaction terminée, on a éliminé l'éthanol à l'évaporateur rotatif, repris dans du chloroforme, séché sur $Na_2SO_4$ anhydre, évaporé le solvant, repris dans l'éthanol absolu et précipité le composé cherché sous forme d'un dichlorhydrate par passage d'un courant d'HCl gazeux sec dans la solution.

On a obtenu 14,7 g de produit ayant pour point de fusion F = 197,2 °C.

Exemple 5

4-[3-(1-propyn-1-yl)-cyclohexyloxy)-2-hydroxy-propyl]-piperazin-1-yl-N-(4-benzoyl-phenyl)-acetamide

En procédant comme indiqué au premier stade de l'exemple 1, mais en partant de 8,2 g (0,072 M) de chlorure de chloracétyle, de 9,5 g (0,048 M) de 4-amino-benzophénone et de 10,1 g (0,096 M) de triéthylamine dissous dans 50 ml de chlorure de méthylène, on a obtenu 11 g de 4-chloroacétamido-benzophénone.

Dans le deuxième stade, on a dissous 5 g (0,018 M) de l'amide précédente dans 100 ml de diméthylformamide, puis on a ajouté 3 g (0,02 M) de la pipérazine monosubstituée utilisée dans les exemples 1 à 3, et 3 g (0,02 M) de carbonate de potassium et laissé la réaction se poursuivre sous agitation à température ambiante pendant 4 heures. On a ensuite repris par de l'eau, extrait à l'éther, lavé, séché sur $Na_2SO_4$ et évaporé à sec, puis repris le résidu par l'alcool à 95 °B et ajouté 4,1 g d'acide maléique en solution dans l'alcool à 95 °B. Le précipité obtenu a été filtré, rincé à l'alcool à 95 °B, puis à l'acétone et à l'éther et séché à 80 °C sous vide.

On a ainsi obtenu 8 g de composé du titre sous forme de dimaléate ayant pour point de fusion F = 126,7 °C.

Exemple 6

4-[3-(α-ethynyl-α-methyl-benzyloxy)-2-hydroxy-propyl]-piperazin-1-yl-N-[2-(2-chlorobenzoyl)-4-chlorophenyl] acetamide

Dans un ballon de 500 ml placé sur agitateur magnétique chauffant, on a introduit 11 g (0,032 M) de 2-chloroacétamido-2', 5-dichloro-benzophénone, 9,2 g (0,032 M) de 1-(α-ethynyl-α-methyl-benzyloxy)-3-piperazin-1-yl-propan-2-ol, 11,3 g (0,112 M) de triéthylamine et 250 ml d'éthanol absolu. Puis on a porté le mélange à reflux pendant 5 heures. La réaction terminée, on a éliminé l'éthanol à l'évaporateur rotatif, repris dans 200 ml de dichlorométhane, lavé la phase organique à l'eau, séché sur $Na_2SO_4$ anhydre, évaporé le solvant, repris dans l'acétone et précipité le dimaléate en ajoutant la quantité nécessaire d'acide maléique à la solution.

On a obtenu 6,5 g de produit ayant pour point de fusion F = 113,5 °C.

Exemple 7

4-[3-(1-(propyn-1-yl)-cyclohexyloxy)-2-hydroxy-propyl]-piperazin-1-yl-N-methyl-N-[4-chloro-2-benzoylphenyl] acetamide

(Composé de l'exemple 2, préparé selon une variante du procédé). Dans un ballon de 1 litre, on a dissous 51,7 g (0,6 M) de pipérazine (base) dans 200 ml de chlorure de méthylène, puis on ajouté par petites fractions 36,7 g (0,1 M) de 5-chloro-2-(N-methyl-bromoacetamido)-benzophenone en maintenant la température inférieure à 30 °C, puis laissé la réaction se dérouler 1 heure sous agitation à température ambiante. On a ensuite lavé la phase organique avec de l'eau, puis séché sur sulfate de sodium, filtré et évaporé sous vide le solvant du filtrat. On a ensuite repris le résidu dans un mélange d'un litre d'eau et 1 litre d'acétone, filtré l'insoluble, extrait par $CH_2Cl_2$, lavé à l'eau, séché puis évaporé. On a ainsi obtenu 14,3 g de 2-[2-piperazinyl-N-methyl-acetamido]-5-chloro-benzophenone.

Dans la deuxième étape, on a dissous 14,3 g du composé précédemment obtenu dans 150 ml d'éthanol absolu, puis on a ajouté 7,5 g de 1-(propyn-1-yl)-3-cyclohexyloxy-1,2-epoxy-propane et maintenu sous agitation 12 heures à température ambiante. On a ensuite évaporé l'alcool et repris le résidu dans de l'acétate d'éthyle puis ajouté 9,1 g d'acide maléique pour obtenir le composé du titre précipitant sous forme de maléate. Après filtration, on a recristallisé le produit dans un mélange éthanol-acétate d'éthyle (1/1), filtré puis séché sous vide à 80 °C. On a ainsi obtenu 13,8 g de produit cherché sous forme de son dimaléate ayant pour point de fusion F = 123,5 °C.

Exemple 8

Préparation de comprimés à 100 mg de principe actif

Chaque comprimé comprend :

| | |
|---|---|
| Composé n° 2 | 100 mg |
| Lactose | 100 mg |
| Amidon | 50 mg |
| Stéarate de Magnésium | 5 mg |

Dans un premier temps, on mélange dans un mélangeur planétaire des quantités égales de composé de l'invention et de lactose, puis on ajoute l'empois d'amidon dans les proportions de la formule centésimale, et on continue à malaxer jusqu'à obtenir une consistance pâteuse. Puis on extrude cette masse pâteuse pour obtenir un granulé qui est ensuite séché, calibré puis concassé.

On ajoute enfin le stéarate dans les proportions de la formule centésimale, puis on confectionne les comprimés dans une presse à comprimer.

Selon les mêmes procédés, on a également préparé d'autres composés de l'invention dont les caractéristiques sont résumées dans le tableau II ci-après.

(Voir Tableau II page 7)

Tableau II

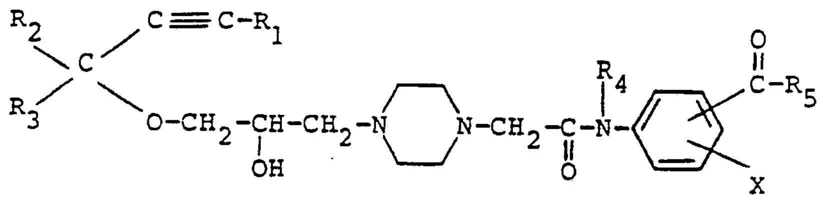

| COMP. N° | PREP. * | $R_1$ | 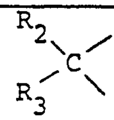 | $R_4$ | 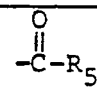 | X | SEL POINT DE FUSION °C |
|---|---|---|---|---|---|---|---|
| 1 | 1 | $-CH_3$ | 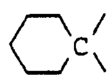 | H | 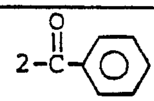 | H | Dimaléate 108,4 |
| 2 | 2 | $-CH_3$ | 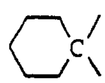 | $-CH_3$ | 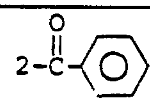 | 4-Cl | Dimaléate 123,6 |
| 3 | 3 | $-CH_3$ | 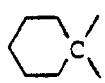 | H | 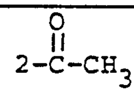 | H | Dimaléate 139,4 |
| 4 | 4 | H | 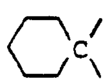 | H | 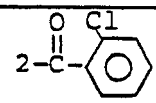 | 4-Cl | Di-HCl 197,2 |
| 5 | 5 | $-CH_3$ | 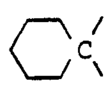 | H | 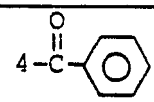 | H | Dimaléate 126,7 |
| 6 | 6 | H | 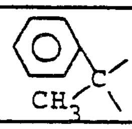 | H | 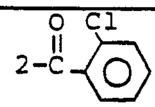 | 4-Cl | Dimaléate 113,5 |
| 7 | 2 | H | 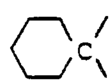 | $-CH_3$ | 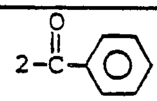 | 4-Cl | Dimaléate 169,3 |
| 8 | 2 | H | 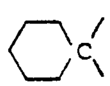 | H | 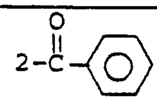 | 4-Cl | HCl 157,5 |
| 9 | 2 | $-CH_3$ | 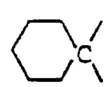 | H | 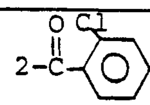 | 4-Cl | Base libre 130,5 |
| 10 | 6 | H | 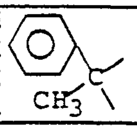 | $-CH_3$ | 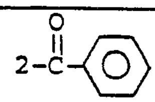 | 4-Cl | Dimaléate 118,9 |

* préparé selon exemple

**Revendications**

1. Composés de formule :

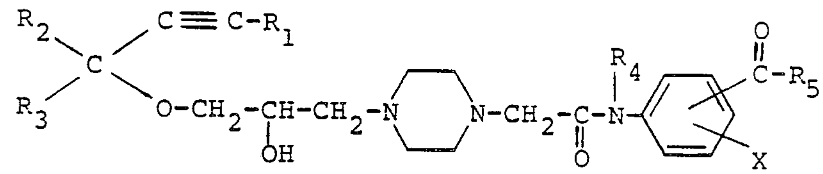

dans laquelle $R_1$ représente l'hydrogène ou un radical alkyle linéaire ou ramifié ayant 1 à 6 atomes de carbone ; $R_2$ et $R_3$ représentent séparément le radical phényle, un radical alkyle linéaire ou ramifié ayant 1 à 6 atomes de carbone, ou ensemble avec l'atome de carbone auquel ils sont liés un radical cycloalkyle de 5 à 7 chaînons ; $R_4$ représente l'hydrogène ou un radical alkyle linéaire ou ramifié ayant 1 à 6 atomes de carbone ; $R_5$ représente un radical alkyle linéaire ou ramifié ayant 1 à 6 atomes de carbone ou le radical phényle éventuellement substitué par un atome d'halogène ; X représente l'hydrogène ou un atome d'halogène et leurs sels d'addition pharmaceutiquement acceptables.

2. Composés selon la revendication 1 dans lesquels $R_1$ à $R_4$ sont des radicaux méthyle.

3. Composés selon la revendication 1 caractérisés en ce que X désigne l'atome de chlore.

4. Composés selon les revendications 1 à 3 caractérisés en ce que le groupe

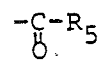

est situé sur le reste phényle en ortho de l'azote amidique.

5. Composé selon la revendication 1 répondant à la formule : 4-[3-(1-propyn-1-yl)-cyclohexyloxy)-2-hydroxy-propyl]-piperazin-1-yl-N-methyl-N-[4-chloro-2-benzoyl-phenyl]-acetamide.

6. Procédé pour la préparation des composés selon l'une quelconque des revendications 1 à 5, caractérisé en ce qu'on fait réagir un composé de formule A, ou un de ses sels

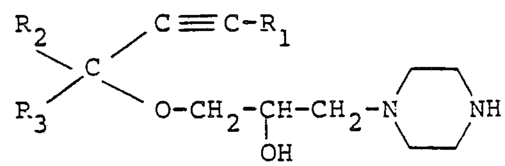

(A)

avec un N-phenyl halogeno-acetamide substitué de formule B

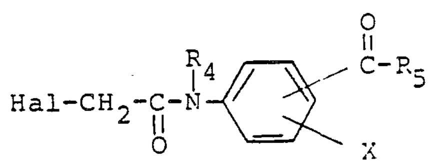

(B)

en présence d'un dérivé basique, les substituants $R_1$ à $R_5$ et X ayant les significations indiquées dans la revendication 1.

7. Procédé selon la revendication 6, caractérisé en ce que le carbonate de potassium ou la triéthylamine est utilisée comme dérivé basique.

8. Procédé pour la préparation des composés selon l'une quelconque des revendications 1 à 5 caractérisé en ce qu'on fait réagir un composé de formule C ou l'un de ses sels

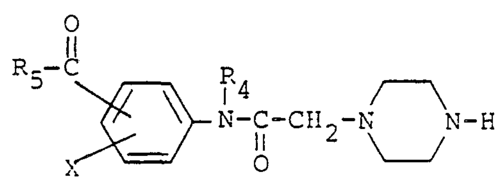

(C)

avec un composé de formule D

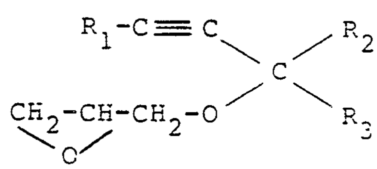

(D)

au sein d'un solvant organique anhydre, les substituants $R_1$ à $R_5$ et X ayant les significations indiquées

dans la revendication 1.

9. Composition pharmaceutique, utile notamment pour le traitement des troubles cardiovasculaires, caractérisée en ce qu'elle comprend, à titre de principe actif au moins un composé selon l'une quelconque des revendications 1 à 5, en association avec des excipients appropriés.

**Claims**

1. Compounds of the formula :

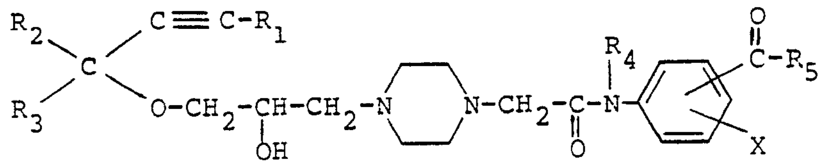

wherein $R_1$ represents hydrogen or an alkyl radical linear or branched having 1-6 C atoms ; $R_2$ and $R_3$ represent separately a phenyl radical, an alkyl radical linear or branched having 1-6 C atoms, or together with the carbon atom to which they are bounded a cycloalkyl radical of 5 to 7 C atoms ; $R_4$ represents hydrogen or an alkyl radical linear or branched having 1-6 C atoms ; $R_5$ represents an alkyl radical linear or branched having 1-6 C atoms or phenyl radical optionally substituted by a halogen atom ; X represents hydrogen or halogen atom, and pharmaceutically acceptable addition salts thereof.

2. Compounds according to claim 1, wherein $R_1$ to $R_4$ are methyl radicals.

3. Compounds according to claim 1 characterised in that X represents a chlorine atom.

4. Compounds according to claims 1 to 3 characterised in that the

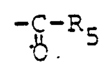

group is situated at the phenyl group in the ortho position with respect to the amido nitrogen.

5. Compound according to claim 1 with the formula : 4-[3-(1-propyn-1-yl)-cyclohexyloxy)-2-hydroxy-propyl]-piperazin-1-yl-N-methyl-N-[4-chloro-2-benzoyl-phenyl]-acetamide.

6. Process for the preparation of the compounds according to anyone of the claims 1 to 5, characterised in that a compound of formula A or their salts thereof

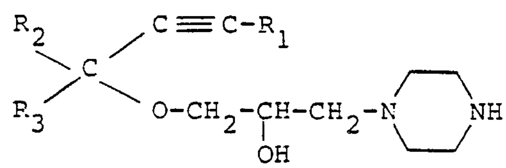

(A)

is reacted with a substituted N-phenyl halogeno-acetamide of formula B

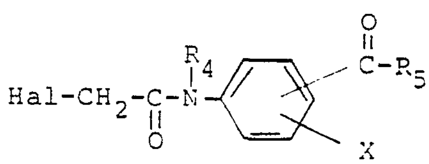

(B)

in the presence of a basic substance, $R_1$ to $R_5$ and X having the same meaning as indicated in claim 1.

7. Process according to claim 6, characterised in that potassium-carbonate or triethylamine is used as basic substance.

8. Process for the preparation fo the compounds according to anyone of the claims 1 to 5 characterised in that a compound of formula C or their salts thereof

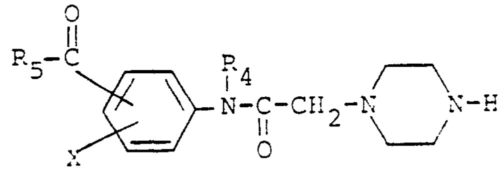

(C)

is reacted with a compound of formula D in an anhydric organic solvent.

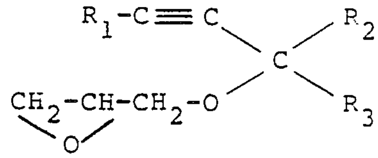

(D)

$R_1$ to $R_5$ and X have the same meaning as indicated in claim 1.

9. Pharmaceutical composition to be used especially in the treatment of cardiovascular disorders comprising as the active principle at least one compound according to anyone of the claims 1 to 5, in combination with suitable carriers.

**Patentansprüche**

1. Verbindungen der Formel :

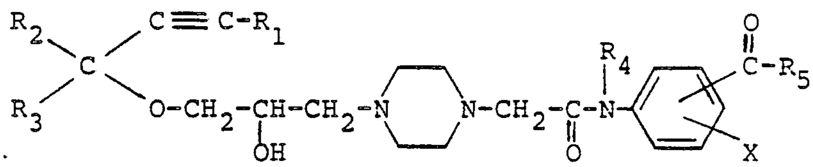

worin $R_1$ für Wasserstoff oder einen geradkettigen oder verzweigten Alkylrest mit 1 bis 6 Kohlenstoffatomen, $R_2$ und $R_3$ getrennt für einen Phenylrest, einen geradkettigen oder verzweigten Alkylrest mit 1 bis 6 Kohlenstoffatomen oder zusammen mit dem Kohlenstoffatom, an das sie gebunden sind, für einen Cycloalkylrest mit 5 bis 7 Ringgliedern, $R_4$ für Wasserstoff oder einen geradkettigen oder verzweigten Alkylrest mit 1 bis 6 Kohlenstoffatomen, $R_5$ für einen geradkettigen oder verzweigten Alkylrest mit 1 bis 6 Kohlenstoffatomen oder einen gegebenenfalls mit einem Halogenatom substituierten Phenylrest und X für Wasserstoff oder ein Halogenatom stehen, sowie deren pharmazeutisch unbedenkliche Additionssalze.

2. Verbindungen nach Anspruch 1, worin $R_1$ bis $R_4$ Methylreste bedeuten.

3. Verbindungen nach Anspruch 1, dadurch gekennzeichnet, dass X ein Chloratom bedeutet.

4. Verbindungen nach Ansprüchen 1 bis 3, dadurch gekennzeichnet, dass sich die Gruppe

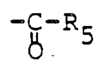

am Phenylrest in der ortho-Stellung zum Amidstickstoff befindet.

5. Verbindung nach Anspruch 1, entsprechend der Formel : 4-[3-(1-Propin-1-yl)-cyclohexyloxy)-2-hydroxypropyl]-piperazin-1-yl-N-methyl-N-[4-chlor-2-benzoylphenyl]-acetamid.

6. Verfahren zur Herstellung der Verbindungen nach einem der Ansprüche 1 bis 5, dadurch gekennzeichnet, dass man eine Verbindung der Formel A oder eines von deren Salzen

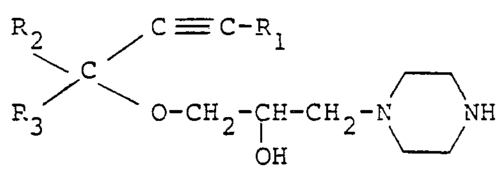

(A)

in Gegenwart eines basischen Derivats mit einem substituierten N-Phenyl-halogenacetamid der Formel B

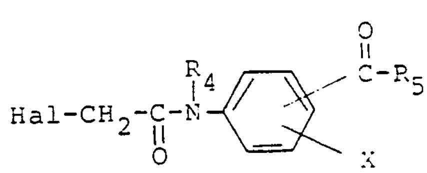

(B)

umsetzt, wobei die Substituenten $R_1$ bis $R_5$ und X die in Anspruch 1 angegebenen Bedeutungen haben.

7. Verfahren nach Anspruch 6, dadurch gekennzeichnet, dass man als basisches Derivat Kaliumcarbonat oder Triäthylamin einsetzt.

8. Verfahren zur Herstellung der Verbindungen nach einem der Ansprüche 1 bis 5, dadurch gekennzeichnet, dass man eine Verbindung der Formel C oder eines von deren Salzen

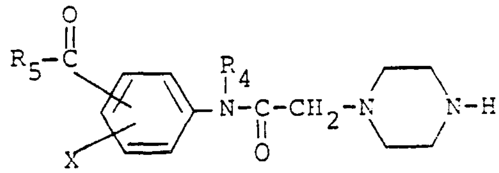

(C)

in einem wasserfreien organischen Lösungsmittel mit einer Verbindung der Formel D

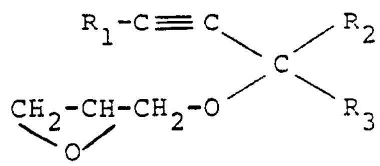

(D)

umsetzt, wobei die Substituenten $R_1$ bis $R_5$ und X die in Anspruch 1 angegebenen Bedeutungen haben.

9. Pharmazeutische Zusammensetzung, insbesondere zur Verwendung bei der Behandlung von Herz Kreislauferkrankungen, dadurch gekennzeichnet, dass sie als Wirkstoff mindestens eine Verbindung nach einem der Ansprüche 1 bis 5 zusammen mit geeigneten Trägern enthält.